# EUROPEAN PATENT APPLICATION

(11) **EP 4 819 154 A1**
(43) Date of publication of application: **07.10.2026**
(21) Application number: 25167639.1
(22) Date of filing: 01.04.2025
(51) Int. Cl.: G16H 40/20, G16H 40/67

(54) **SYSTEM AND METHOD FOR ASSIGNING A MEDICAL DEVICE TO A SPECIFIC LOCATION WITHIN A HEALTHCARE FACILITY**

(71) Applicant: B. Braun Melsungen AG, 34212 Melsungen (DE)
(72) Inventor: BUERGER, Maria, 34323 Malsfeld (DE); STEUBE, Johannes, 34593 Knüllwald (DE); SCHWARZ, Jan, 34212 Melsungen (DE)
(74) Representative: Tergau & Walkenhorst Intellectual Property GmbH

(57) **Abstract**

Main claim: A system for assigning a medical device (2) to a specific location within a healthcare facility (4), the system comprising:
a) at least one medical device (2), having:
• a user interface (6) configured to allow selection of a predefined location within a hierarchical healthcare facility structure;
• a memory (8) for storing an assigned location information; and
• a communication module (12) configured to exchange the location information with other medical devices (2') and/or a server (10);

b) a server (10) or decentral/distributed configuration device configured to:
• store and manage said hierarchical healthcare facility structure, including in particular locations ranging from facility level to individual beds;
• receive and synchronize location assignments from the medical device (2); and
• distribute updated location information to other medical devices (2'),

c) a communication network (14) interconnecting the medical device(s) (2, 2') and the server (10) or decentral configuration device.

## Description

### Technical Field

The present invention relates to medical technology and, more particularly, to systems and methods for assigning medical devices to specific locations within a healthcare facility. The invention ensures that medical devices, such as infusion pumps, can be accurately and efficiently associated with their respective locations in a structured manner, facilitating improved device management and patient safety.

### Background Art

In modern healthcare environments, medical devices are frequently moved between different locations, such as patient rooms, intensive care units, and operating theaters. However, accurately tracking and managing these devices within a hospital is a challenge. Conventional approaches often rely on manual logging, barcode scanning, or radio-frequency identification (RFID) tags, which can be inefficient and error-prone. Moreover, the lack of real-time synchronization between devices and hospital information systems (HIS) can lead to discrepancies in device allocation and patient association, potentially compromising patient care.

Existing systems do not adequately provide a seamless and automated way to assign medical devices to specific locations in a structured and hierarchical manner. Furthermore, they fail to facilitate efficient synchronization of location assignments across multiple devices, limiting the potential for improved hospital workflows and patient safety.

More specifically, existing solutions such as RFID, Wi-Fi triangulation, and ADT data systems have notable drawbacks that the present invention addresses. RFID tags and Bluetooth beacons, while useful for real-time tracking, are often costly and require significant implementation efforts. Moreover, they do not display location information directly on the medical device's user interface, making it less intuitive for users. Wi-Fi triangulation, although utilizing existing infrastructure, often lacks the precision needed to pinpoint the exact location of medical devices, which can lead to inefficiencies in device management. ADT systems, integrated with Patient Data Management Systems (PDMS), track patient-related information but fail to provide specific location data for the medical devices, limiting their usefulness for precise device tracking.

### Object of the Invention

It is an object of the present invention to provide an improved system and method for assigning medical devices to specific locations within a healthcare facility. The invention aims to reduce errors associated with manual tracking, enhance device management through structured location assignment, and enable seamless synchronization of location information among multiple medical devices and hospital systems.

### Solution to the Problem

To address the aforementioned problems, the invention provides a system for assigning a medical device to a specific location within a healthcare facility. According to claim 1, the system comprises:
- at least one medical device with a user interface for selecting a predefined location, a memory for storing assigned location information, and a communication module for exchanging the location information with other medical devices and/or a server;
- a server or decentralized/distributed configuration device for managing a hierarchical healthcare facility structure, receiving and synchronizing location assignments, and distributing updated location information; and
- a communication network interconnecting the medical device(s) and the server or decentralized configuration device.

Additionally, the invention provides a method for assigning a medical device to a specific location. According to claim 10, the method includes:
- defining a hierarchical healthcare facility structure;
- storing this structure in a server and/or medical device;
- enabling a user to select a location via a user interface;
- storing the assigned location in the medical device's memory;
- transmitting and synchronizing the assigned location information with a server and/or other medical devices.

Together, these features ensure accurate device location assignment and synchronization, reducing manual tracking errors and enhancing hospital workflow efficiency.

The objectives, features, embodiments, and advantages described within this text for the system also apply correspondingly to the method, and vice versa. Specifically, the use of communication interfaces and protocols, pre-filtering mechanisms, vicinity detection, short code assignment, patient confirmation, and controlled update distribution enhance both the system and the method, providing a comprehensive and flexible solution for medical device location assignment within a healthcare facility.

### Advantageous Embodiments

Various advantageous embodiments to be discussed below further improve the system and method:
In a preferred embodiment, the system utilizes a server configuration, where the hierarchical healthcare facility structure and location data are stored and managed centrally. However, in alternative embodiments, a decentralized or distributed configuration device can be used, where the hierarchical structure and location information are stored locally on each device or distributed across multiple devices. This allows for flexibility in scalability and reduces the dependency on a single central server, making the system more resilient to network failures and providing operational independence.

Advantageously, the communication module of the medical device is configured to utilize at least one of the following communication interfaces: Wi-Fi, Bluetooth, Infrared (IrDA), or RFID. These interfaces enable flexible and reliable communication in different hospital environments.

In a preferred embodiment, data exchange between the medical device and the server and/or other medical devices and/or the configuration device is performed using at least one of the following communication protocols: HL7, SDC, TCP/IP, HTTP, HTTPS**,** or a proprietary protocol. These protocols ensure interoperability with existing hospital IT systems and secure data transmission.

Advantageously, the medical device comprises a filtering mechanism configured to pre-filter possible location assignments based on detected MAC addresses or other network-related identifiers of nearby access points or on vicinity information derived from short-range communication with other devices. This reduces the number of possible locations presented to the user, simplifying and accelerating the assignment process.

In a preferred embodiment, the medical device automatically analyzes or detects its vicinity based on received Bluetooth signals, shared SSIDs, or physical proximity in a shared docking station. This enables automatic or semi-automatic location assignment without user intervention.

Advantageously, a number of, in particular each, predefined location(s) within the healthcare facility structure is associated with a unique short code, which can be manually entered into the medical device for quick location assignment. This feature simplifies manual entry and reduces the likelihood of input errors.

In a preferred embodiment, the user interface prompts the user to confirm a link between the assigned location, the medical device, and a patient before initiating medical treatment. This ensures patient safety by preventing mismatches between devices and patients.

Advantageously, the updated location information is distributed to other medical devices within a predefined vicinity level only, such as within the same hospital unit, room, or docking station. This reduces unnecessary data traffic and focuses updates only on relevant devices.

In a preferred embodiment, the medical device is selected from the group comprising infusion pumps, patient monitors, ventilators, dialysis machines, syringe pumps, anesthesia machines, defibrillators, respiratory devices, or other electronically controlled portable medical devices used in a healthcare facility. This ensures broad applicability of the invention across various medical fields.

Advantageously, the medical device determines its approximate location based on MAC addresses from nearby access points or proximity information from short-range communication with other devices and displays a reduced list of possible locations in the user interface. This simplifies location assignment by showing only the most likely locations, reducing the risk of errors and improving efficiency, especially for mobile devices that move within the healthcare facility.

In a preferred embodiment, the medical device synchronizes location assignments with a hospital information system (HIS) to integrate them into electronic medical records. This centralizes the management of device locations, ensuring accurate tracking and enabling quick access to device data, especially in emergencies. It also helps integrate device locations directly into patient records, reducing the risk of misplacements or errors.

Advantageously, the medical device receives location updates from nearby devices via short-range communication, such as Bluetooth or Infrared (IrDA). This automatic adjustment of device locations ensures accuracy without requiring manual input, particularly in settings where devices are frequently moved between rooms or units.

The use of short-range communication ensures only nearby devices exchange data, preventing erroneous location assignments.

In a preferred embodiment, the medical device displays the assigned location information on its user interface for verification by medical personnel. This adds an extra layer of safety, allowing personnel to confirm the correct location before the device is used, reducing the risk of location errors and increasing the trust in the system.

Advantageously, location information is distributed only to other medical devices within a predefined proximity, such as within the same hospital unit, room, or docking station. This selective distribution ensures that only relevant devices receive location data, preventing unnecessary network load and maintaining efficient device management within a designated area.

### Additional Information

The invention described here provides a procedure for assigning medical devices, such as infusion pumps, to specific areas within healthcare facilities. The underlying healthcare facility structure, also called hospital structure, can be defined in an associated software (stored in each medical device and/or as a server application) and either retrieved online from the device or stored as a file. Users can select the location on the device's user interface (UI), assign it accordingly, and this information is mirrored back to the software and synchronized across all devices.

More specifically, in an associated software for the medical device, which is part of each medical device or/and part of a server application, users can define the hospital structure in a hierarchical manner, starting from the highest level (the facility) down to the most granular level (individual beds). The detailed hospital structure (highest level to lowest level) is usually: facility, building, floor, room, individual bed. This detailed structure can be created and managed within the software (in the device and/or the server application), ensuring that every area of the hospital is accurately represented.

There are basically two ways to handle this hospital structure:
1. **Server Retrieval:** The structure can be dynamically retrieved from an IT server (server application). This ensures that the most up-to-date information is always available.
2. **Local Storage:** Alternatively, the structure can be stored as a file directly on the medical device. This allows for offline access and ensures that the device can function independently of a server connection. Local storage also allows for synchronization with other medical devices if the structure has been changed on the medical device.

Once the hospital structure is defined, users can interact with the device's user interface (UI) to select the specific location where the device will be used. For example, a user can assign an infusion pump to a particular bed in a specific ward (floor). This assignment process is intuitive and user-friendly, allowing for quick and accurate device allocation.

After the location is selected and assigned on the medical device, this location information is mirrored back to the associated software at the server application. This means that any changes made on the device are automatically updated in the software at the server application, ensuring consistency and accuracy across the system.

Furthermore, the location information is distributed and synchronized to all relevant medical devices at the bed level, i.e. the information is also distributed to medical devices that are in the vicinity of the medical device where the changes (location assignment) have been made. This synchronization can be achieved through various communication techniques: various interfaces, including:
- **Wi-Fi:** a wireless communication method that allows devices to exchange information over short distances.
- **IrDA:** a communication method via infrared to share the data across all devices such as infusions pumps inserted into an associated rack or station.
- **Bluetooth:** a short-range wireless technology standard that is used for exchanging data between fixed and mobile devices over short distances.
- **Radio-frequency identification (RFID):** uses electromagnetic fields to automatically identify and track tags attached to objects. It consists of a tiny radio transponder called a tag, a radio receiver, and a transmitter.

Possible communication protocols are as follows:
- **SDC (Service-oriented Device Connectivity):** a standard for medical device interoperability that facilitates seamless communication between devices.
- **HL7 (Health Level 7):** a set of international standards for the exchange of electronic health information, ensuring that devices can communicate effectively within the healthcare environment.
- **NFC (Near-Field Communication):** is a set of communication protocols that enables communication between two electronic devices over a short distance.
- **HTTP (Hypertext Transfer Protocol) and HTTPS (Secure Hypertext Transfer Protocol):** is an application layer protocol in the Internet protocol suite model for distributed, collaborative, hypermedia information systems.
- **TCP/IP (Internet protocol suite):** is a framework for organizing the set of communication protocols used in the Internet and similar computer networks according to functional criteria.
- **Protocol Buffers (Protobuf):** is a free and open-source cross-platform data format used to serialize structured data
- **Proprietary protocols:** any specific communication protocol that does not follow any standardized communication protocol.

Additionally, the system includes a pre-filtering feature (e.g. based on filtering by MAC addresses) or entering short code location. This allows for pre-filtering of data based on MAC addresses that have been received from an access point in the vicinity of the medical device. The idea is to map rooms/locations to single access points and thus assuming that the medical devices that are connected to this access point are in the same room/location. The list of MAC address is used for selecting the respective medical devices (automatically or manually and allows to cluster the individual devices to one room/location), i.e. the MAC-address of the medical device connected to one access point is the filter criteria to pre-filter/limit the number of possible medical devices at one location.

An alternative pre-filtering approach can be realized when the devices in a vicinity know each other, e.g. by similar SSIDs or Bluetooth signals received between some devices in a close area, or if the medical devices are attached to the same Station/Rack. Those technologies also allow to group the medical device and to assign them to a location/room. That is, the pre-filtering method assumes that the medical devices in a close vicinity know each other (and probably also some location information) and thus limit the number of medical devices selected for one location/room.

The optional short-code location-approach allows the operator to enter quickly a location assignment using short codes (e.g. 'AB1', 'Bed02', 'B02W', etc.). Preferably, any location has a short code, e.g. 'AB1', to make the location entry much easier and faster. Any code is unique in the hospital structure. This method enhances the efficiency and accuracy of device management.

This approach allows to connect the patient, the pump, and the location where the pump is been used. All those information are preferably stored on the server and visualized on the device UI. In case of an infusion pump, during the infusion programming process, the user needs to confirm / validate the connection between patient, pump and location. When the infusion is started, all those data are continuously transferred to the server and available for the hospital depending on the HIS (Hospital information system) and thus used for the electronic medical record or being displayed on other medical devices such as monitoring. For any service activities, the selected location might help for identifying pump's location.

In an enhanced mode, the assigned location also limits the number of possible therapies.

This comprehensive approach ensures that all medical devices are accurately tracked and managed, improving operational efficiency and patient care within healthcare facilities.

The healthcare facility can refer to various medical environments where patient care is provided. This includes hospitals, ranging from large multi-building medical centers to smaller regional or specialized hospitals. It also encompasses clinics, such as outpatient care centers, dialysis clinics, or specialized treatment facilities. Additionally, the term covers long-term care facilities, including nursing homes and rehabilitation centers, as well as emergency care units like ambulances or field hospitals.

### Summary of the Invention

In summary, the present invention provides a system and method for efficiently assigning medical devices to specific locations within a healthcare facility. The invention ensures structured location management, reduces errors in device tracking, and improves integration with hospital IT systems. By leveraging various communication interfaces and protocols, filtering mechanisms, and automated detection techniques, the invention significantly enhances the accuracy and efficiency of medical device management in hospital environments.

Furthermore, the system enables additional functionalities based on the assigned location of the medical device. For instance, the medical device can automatically adjust its settings according to the assigned care unit, ensuring that predefined parameters such as infusion rates or medication protocols are in line with the specific requirements of that unit. This automatic configuration minimizes the risk of human error and enhances the efficiency of medical workflows.

Additionally, the system allows for the synchronization of patient-related data with hospital information systems. Upon assignment of a medical device to a specific location, relevant patient information, such as patient ID, weight, gender, or treatment protocols, can be retrieved and associated with the device. This linkage ensures seamless integration with electronic medical records, improving data accuracy and facilitating informed clinical decision-making.

Moreover, the invention enhances the accuracy of location assignment by leveraging surrounding network information. Medical devices in the vicinity can be detected through shared network parameters such as access points, MAC addresses, or Bluetooth signals. This information is used to refine the selection of possible locations, reducing the number of options presented to the user and thereby streamlining the assignment process. The system thus supports both manual assignment via short codes and automated filtering based on environmental data, offering a flexible and reliable solution for healthcare facilities.

### Brief Summary of the Figures

Exemplary embodiments of the invention are subsequently discussed with reference to the accompanying drawings.
FIG. 1 illustrates a schematic overview of a system according to the invention, illustrating the assignment of a medical device, here represented by an infusion pump, to a specific location within a healthcare facility.
FIG. 2 displays an example of the user interface for the infusion pump system, showing the "Patient Assignment" menu with input/display fields for Patient ID, Care Unit, Hospital, and Bed Number.
FIG. 3 presents another example of the user interface for the infusion pump system, displaying the same elements as in FIG. 2 but arranged differently for overview.
FIG. 4 illustrates a hierarchical tree diagram representing the relationship between the physical structure and the organizational structure of a healthcare facility.

### Detailed Description of at preferred Embodiment

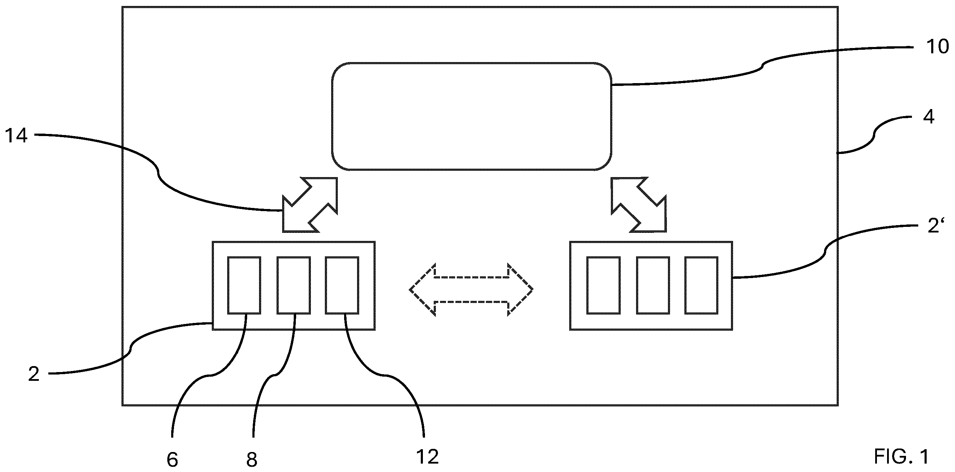

FIG. 1 shows an exemplary system for assigning a medical device to a specific location within a healthcare facility.

The system includes at least one medical device 2, such as an infusion pump, which is assigned to a specific location within the healthcare facility 4. The location indication is given with respect to a predefined hierarchical healthcare facility structure which may range from a general facility level to a more granular level, such as individual rooms or even patient beds. The medical device 2 includes a user interface 6 that allows the user to select a predefined location from such a hierarchical structure of the healthcare facility 4, which structure is stored in memory 8 within the medical device 2 itself or externally in a server 10. The communication module 12 of the medical device 2 enables the device to exchange location information with other medical devices 2' and/or a server 10, which is connected via a communication network 14.

The server 10, or alternatively a decentralized configuration device, is responsible for storing and managing the hierarchical healthcare facility structure, which preferably includes the details of locations ranging from the facility level to individual beds. This server 10 also receives and synchronizes location assignments from the medical device 2, ensuring that all devices within the network have up-to-date location data. The communication network 14 interconnects the medical device 2 with the server 10 or configuration device 10, allowing data to be shared efficiently across the system.

The hierarchical healthcare facility structure can be represented in various ways within the data structure and software of the medical devices. A tree structure allows hierarchical organization, linking each level (e.g., hospital → building → floor → room → bed). A relational database stores levels in separate tables with unique identifiers for efficient queries. A key-value store enables fast lookups, while a graph-based approach supports complex relationships, such as devices associated with multiple locations. Alternatively, a flat list with encoded hierarchy (e.g., "HOSP1-BLD3-FL2-RM12-BED4") provides a memory-efficient format. Data can also be exchanged in JSON or XML, ensuring interoperability. The choice depends on system requirements, performance needs, and hospital infrastructure compatibility.

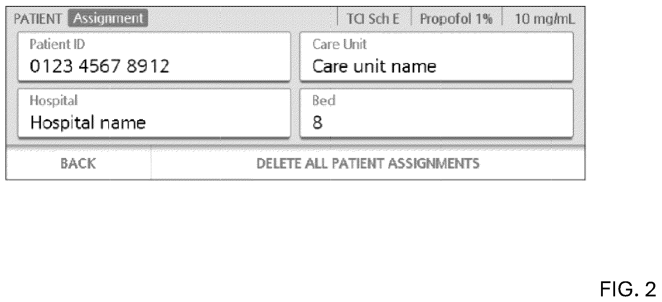

FIG. 2 illustrates an example of the user interface 6 of an infusion pump, representing a medical device 2, where a menu titled "Patient Assignment" is displayed. The user interface allows the user to either manually input or select the patient's ID (e.g., "0123 4567 8912") from a dropdown menu. Alternatively, the patient-ID may be preassigned by a server and requires user confirmation. Additionally, the interface enables the selection of a care unit (e.g., "ICU") and the assignment of the device to a specific bed number (e.g., "8") within the healthcare facility. By providing these functionalities, the user interface 6 facilitates the efficient and error-free assignment of the medical device 2, ensuring proper linkage with the correct patient and care unit while allowing for both user-driven and system-guided assignments.

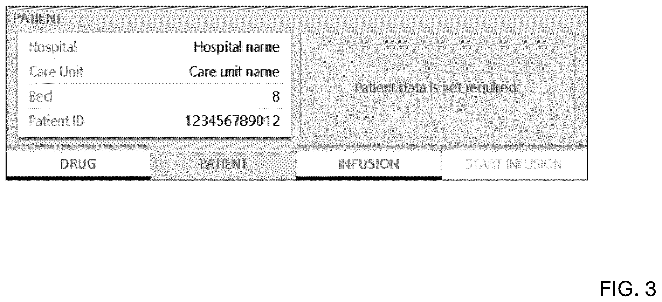

FIG. 3 shows a further example of the user interface 6 arranged in a different format for better overview and ease of use. The same input fields are provided as in FIG. 2, including the Patient ID, Care Unit, and Bed number in terms of a more detailed user interface layout.

In this embodiment, the communication module 12 of the infusion pump 2 is configured to use communication protocols such as Wi-Fi, Bluetooth, or Infrared (IrDA) to exchange data between medical devices 2 and the server 10. The data exchange is conducted using standardized communication protocols such as HL7, SDC, TCP/IP, or proprietary protocols to ensure smooth and secure transmission of location data. The system is capable of pre-filtering possible location assignments based on proximity data derived from detected MAC addresses or other network-related identifiers, helping to reduce errors during the location assignment process.

Furthermore, the system can be designed to automatically detect the medical device's vicinity based on Bluetooth signals, shared SSIDs, or physical proximity in a docking station, thus simplifying the location assignment process. The hierarchical structure of the healthcare facility is stored within the memory 8 of the medical device 2 and/or externally on the server 10, allowing for efficient location assignment even in large healthcare facilities.

The memory 8 within the medical device 2 stores the assigned location information, which can be updated and transmitted to other medical devices 2' or the server 10 for synchronization. In this manner, the medical device 2 ensures that it is always aligned with the correct location data, which can be crucial in providing safe and efficient medical care.

In a preferred embodiment, the medical device 2 displays the assigned location information on its user interface 6, allowing medical personnel to verify the information before initiating medical treatment. This ensures that the correct device is assigned to the correct location and patient, minimizing the risk of errors. The system can also limit the distribution of location information to other medical devices 2' within a predefined vicinity, such as within the same care unit, room, or docking station, to ensure that only relevant devices receive updates.

In a variation, the medical devices 2 are capable of forming an ad-hoc network, allowing direct communication and synchronization of location information without requiring constant connection to the server 10. This enables medical devices 2 within a predefined vicinity to exchange data efficiently, even in cases where network infrastructure is limited or temporarily unavailable. The ad-hoc network can be established using short-range communication technologies such as Bluetooth, Infrared (IrDA), or other peer-to-peer wireless protocols, ensuring seamless integration and coordination among medical devices 2 within the healthcare facility.

In summary, this system provides a streamlined and efficient method for assigning and managing the locations of medical devices 2 within a healthcare facility 4. The ability to automatically detect and update location data through the use of communication protocols, filtering mechanisms, and a hierarchical facility structure ensures that medical devices 2 are always properly assigned, leading to improved efficiency and reduced errors in healthcare environments.

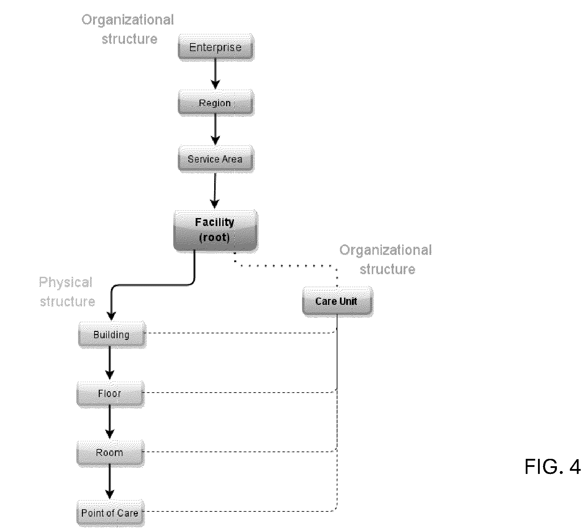

FIG. 4 illustrates a hierarchical tree diagram representing the relationship between the physical structure and the organizational structure of a healthcare facility. The physical structure includes elements such as buildings, floors, rooms, and individual beds, while the organizational structure defines units such as departments, care units, and specialized treatment areas. The figure demonstrates how medical devices can be assigned within these structures to ensure accurate location tracking and integration with hospital management systems.

### List of reference numerals

- 2, 2': medical device
- 4: healthcare facility
- 6: user interface
- 8: memory
- 10: server
- 12: communication module
- 14: communication network

## Claims

1. A system for assigning a medical device (2) to a specific location within a healthcare facility (4), the system comprising:
a) at least one medical device (2), having:
• a user interface (6) configured to allow selection of a predefined location within a hierarchical healthcare facility structure;
• a memory (8) for storing an assigned location information; and
• a communication module (12) configured to exchange the location information with other medical devices (2') and/or a server (10);
b) a server (10) or decentral/distributed configuration device configured to:
• store and manage said hierarchical healthcare facility structure, including in particular locations ranging from facility level to individual beds;
• receive and synchronize location assignments from the medical device (2); and
• distribute updated location information to other medical devices (2'),
c) a communication network (14) interconnecting the medical device(s) (2, 2') and the server (10) or decentral configuration device.

2. The system according to claim 1, wherein the communication module (12) of the medical device (2) is configured to utilize at least one of the following communication interfaces: Wi-Fi, Bluetooth, Infrared (IrDA), or RFID.

3. The system according to claim 1 or 2, wherein the medical device (2) is configured to synchronize patient-related data, including at least one of patient-ID, weight, gender, and height, with a hospital information system (HIS) based on the assigned location, and/or to automatically adjust operational settings of the medical device (2) according to location-specific parameters.

4. The system according to any one of the preceding claims, wherein the medical device (2) comprises a filtering mechanism configured to pre-filter possible location assignments based on signals received from other devices, such as detected MAC addresses or other network-related identifiers of nearby access points, or on vicinity information derived from short-range communication with other devices (2').

5. The system according to any one of the preceding claims, wherein the medical device (2) automatically analyzes or detects its vicinity based on signals received from other devices, such as received Bluetooth signals, shared SSIDs, or physical proximity in a shared docking station.

6. The system according to any one of the preceding claims, wherein a number of, in particular each, predefined location(s) within the healthcare facility structure is associated with a unique short code, which can be manually entered into the medical device (2) for quick location assignment.

7. The system according to any one of the preceding claims, wherein the user interface (6) prompts the user to confirm a link between the assigned location, the medical device (2), and a patient before initiating medical treatment.

8. The system according to any one of the preceding claims, wherein the updated location information is distributed to other medical devices (2') within a predefined vicinity level only, such as within the same hospital unit, room, or docking station.

9. The system according to any one of the preceding claims, wherein the medical device (2) is selected from the group comprising infusion pumps, patient monitors, ventilators, dialysis machines, syringe pumps, anesthesia machines, defibrillators, respiratory devices, or other electronically controlled portable medical devices used in a healthcare facility (4).

10. A method for assigning a medical device (2) to a specific location within a healthcare facility (4), the method comprising:
a) defining a hierarchical healthcare facility structure, including in particular a facility, building, floor, room, and/or individual bed;
b) storing said hierarchical healthcare facility structure in a server (10) and/or the medical device (2);
c) allowing a user to select a location for the medical device (2) via a user interface (6) based on said hierarchical healthcare facility structure;
d) storing an assigned location information in a memory (8) of the medical device (2);
e) transmitting and updating the assigned location information to a server (10) and/or synchronizing it with other medical devices (2').

11. The method according to claim 10, wherein the medical device (2) determines its approximate location based on MAC addresses received from nearby access points or vicinity information derived from short-range communication with other medical devices (2') and provides a reduced list of possible locations in the user interface (6).

12. The method according to claim 10 or 11, wherein the medical device (2) synchronizes location assignments with a hospital information system (HIS) for integration into electronic medical records.

13. The method according to any one of claims 10 to 12, wherein the medical device (2) receives location updates from other medical devices (2') in the vicinity via short-range communication, such as Bluetooth or Infrared (IrDA).

14. The method according to any one of claims 10 to 13, wherein the medical device (2) displays the assigned location information on its user interface (6) for verification by medical personnel.

15. The method according to any one of claims 10 to 14, wherein the location information is distributed to other medical devices (2') within a predefined vicinity level only, such as within the same hospital unit, room, or docking station.
